# EUROPEAN PATENT APPLICATION

(11) **EP 2 172 256 A1**
(43) Date of publication of application: **07.04.2010**
(21) Application number: 08169069.5
(22) Date of filing: 13.11.2008
(51) Int. Cl.: B01D 53/84, C12M 1/04

(54) **Device for oxidation of methane in residual biogas**

(30) Priority: 03.10.2008 PL 38621008
(71) Applicant: Politechnika Lubelska, 20-219 Lublin (PL)
(72) Inventor: Pawlowski, Lucjan, 20-027, LUBLIN (PL); Pawlowska, Malgorzata, 20-027, LUBLIN (PL)
(74) Representative: Kaminski, Piotr

(57) **Abstract**

A device for oxidation of methane in residual gas from a waste dump comprising waste dump degassing well and biofilter is **characterised in that** well (1) with waste dump gas is connected with biofilter (3) by a pipe (2) having a diameter equal to the diameter of the well (1), which is connected with at least one pipe (2a), having along its entire length, holes (4) having a section area selected in such a way, that the total area of the section of all holes (4) is equal to the internal area of the pipe (2a). Above the pipe (2a), in the biofilter (3) there is a grate (5) made of net with mesh diameter of 0,5 cm and on the grate (5) there is a layer, 5-15 cm thick, preferably 10 cm thick, of keramsite having a granularity of 0,6 to 1,2 cm, and above the keramsite layer (6) there is a 50-80 cm high layer of organic material, preferably a layer of compost, in which layer methane oxidising bacteria can develop. The waste dump gas present in the biofilter in the space beneath the grate (5) attains constant overpressure, the same as that in the degassing well (1), which ensures its uniform inflow to the entire surface of the layers (6) and 7) in the biofilter (3)

## Description

The subject of this invention is a device for oxidation of methane in residual gas from a waste dump - biogas.

In non-segregated waste dump methane is produced in the process of methane fermentation. In the most beneficial period of methanogenesis the gas from the waste dump contains around 70% of CH₄ and 30% of CO₂. The energy value of such gas is high and it is in common use as s source of energy. After several years the intensity of methane production in the waste dump drops and the methane content in the waste dump gas drops below 30%. It has then too low an energy value to be exploited for energy purposes.

Emission of methane to the atmosphere is undesirable, for methane is one of the important greenhouse gases. It absorbs infrared radiation 20 times more than commonly known carbon dioxide (as per number of molecules). The need for acting against greenhouse effect requires that emission of methane to the atmosphere be limited.

One of the methods of biogas treatment which leads to elimination of methane emission to the atmosphere involves removal of CO₂ through freezing out the low methane content biogas. As a result biogas containing more than 30% of methane is obtained and as such it can be utilised for energy purposes. This method however involves high energy consumption. This drawback does not apply to methods based on biological oxidation of methane by methanotrophs. Biological oxidation can be carried out in old waste dumps, not covered with membrane.

In case of membrane covered waste dumps the biogas is carried away through specially situated degassing wells. In this case it is possible to oxidise methane in specially designed biofilters.

British patent GB1150401 describes a method of methane oxidation in biofilter filled with a solution of a suspension containing methane oxidising bacteria and nutrients in following concentrations: 0,6 g Na₂HPO₄/dm³, 0,4 g KH₂PO₄/dm³, 0,1 g NH₄Cl/dm³, 0,2 g MgSO₄ x 7H₂O/dm³, traces of ions of bio-elements such as Fe, Ca, Zn, Cu, Mn. According to inventors of the cited patent it is possible to oxidise methane even at the flow speed of 0,12 m³ of gas per m³ of the solution.

Chinese patent CN 101085693 presents a design of a bioreactor to which biogas is delivered from down and where it first goes through a porous layer formed from sawdust in order to ensure the uniform flow through the entire reactor. Then the gas enters into reactive layer of granulate containing methane oxidising bacteria.

In another Chinese patent CN101016512 a reactor is presented, where the biogas is introduced from down and then it goes through a bed containing methanotrophs. Along the length of the reactor, at certain distances, air is injected, which according to inventors ensures uniform methane oxidation in the entire bed.

Despite application of porous layer formed in the lower part of reactor above the pipe carrying away the biogas to biofilter, it is difficult to ensure uniform flow, because most of the degassing wells have diameters between 20 and 60 cm. Meanwhile to ensure complete oxidation of biogas emitted through one degassing well, the section of the biofilter should have an area of several to even more than ten square meters. Given such a large difference in sections, the biogas tends to flow faster upwards in the area located directly above the delivering pipe.

The essence of the device for oxidation of methane in residual gas from a waste dump comprising a waste dump degassing well and biofilter is that a well with biogas is connected with biofilter by a pipe having a diameter equal to the diameter of the well, which is connected with at least one pipe, preferably several located in a distance of around 50 cm one from another, passing above the bottom of the biofilter, tangent to its horizontal section and having from down, along its entire length, holes, located in such a way, that the whole segment of the pipe located inside the biofilter is divided into five equal segments, the length of each being equal to 20% of the length of the pipe, and in the consecutive segments holes are made in the following order: on certain segment, starting from biogas inlet, there is one row of holes, on the second segment there are two rows, on the third segment there are three rows, on the forth - four rows and on the fifth segment there five rows of holes having section selected in such a way, that the total area of the section of all holes is equal to the internal area of the pipe. Above the pipe, in the biofilter there is a grate made of net with mesh diameter of 0,5 cm and on the grate there is a keramsite layer, 5-15 cm thick, preferably 10 cm thick, having a granularity of 0,6 to 1,2 cm, and above the keramsite layer there is a 50-80 cm, preferably 70 cm high layer of organic material, preferably a layer of compost, in which layer methane oxidising bacteria can develop.

Biogas present in the biofilter in the space beneath the grate attains constant overpressure, the same as that in the degassing well, which ensures its uniform inflow to the entire space in the biofilter.

The beneficial effect of the device according to the invention is that it allows for uniform flow of waste dump gas in the entire section of the biofilter, which is especially important when there is a big difference between the section of the pipe delivering the waste dump gas and the section of biofilter.

An embodiment of the device is presented in schematic drawing.

The waste dump gas is delivered through a pipe 2 and one or several pipes 2a of the same diameter as that of degassing well 1, introduced in parallel to the horizontal section of biofilter 3, to its lower part situated above the bottom of biofilter 3, at the height of about 15 cm, separated from remaining layers of biofilter 3 with a grate 5 which allows for creation of space free from filling of biofilter 3.

On a part of pipe 2a delivering the waste dump gas, located inside the biofilter 3, on its bottom wall, holes 4 are made and situated in such a way, that on each subsequent segment of the pipe 2a, located inside the biofilter and equalling 20 % of its length, there are holes 4 so located , that on certain segment, starting from inlet of waste dump gas there is one row of holes, on the second there are two rows, on the third - three rows, on the fourth - four rows and on the fifth there are five rows having area selected in such a way, that the total area of the section of all holes 4 is equal to the internal area of the pipe 2a.

This ensures uniform inflow of the gas into entire surface of biofilter 3, which is because of uniform overpressure created in the space free from filling of biofilter 3. Above the grate 5 which supports the filling of the bed two layers are formed, a drainage layer 6, 5-15 cm thick, preferably 10 cm, composed of keramsite of granularity of 0,6-1,2 cm, a second layer 7, 50-80 cm high, preferably 70 cm, composed of organic material, preferably compost, in which methanotrophs can develop.

Waste dump gas from well 1 is delivered by pipes 2 and 2a, having a diameter equal to diameter of degassing well 1, in such a way, that the pipe 2 delivering waste dump gas is introduced just above the bottom of biofilter 3, from the side, tangental to its horizontal section, and on its entire lengths from down holes are made and located in such a manner that on the first segment, starting from inlet of waste dump gas there is one row of holes, on the second there are two rows, on the third - three rows, on the fourth - four rows and on the fifth there are five rows having internal area so selected, that the total area of the section of all holes 4 is equal to the internal area of the pipe 2a. Above the pipe 2a there is a grate 5 made of net with mesh diameter of 0,5 cm. On the grate 5 there is a 5-15 cm thick, preferably 10 cm thick layer of keramsite 6 of granularity of 0,6-1,2 cm, and above keramsite layer 6 there is a layer 7, 50-80 cm thick, preferably 70 cm, composed of compost or compost mixed with bark in which methane oxidising bacteria can develop.

Waste dump gas in the space beneath the grate attains constant overpressure, the same as that in the degassing well, which ensures its uniform flow through the bed.

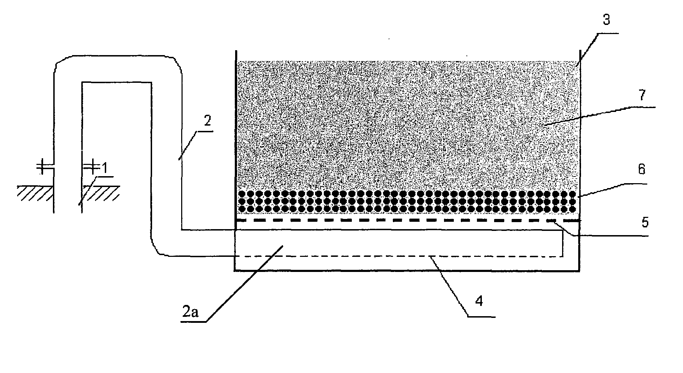

## Claims

1. A device for oxidation of methane in residual gas from a waste dump comprising waste dump degassing well and biofilter **characterised in that** a well (1) with waste dump gas is connected with biofilter (3) by a pipe (2) having a diameter equal to the diameter of the well (1), which is connected with at least one pipe (2a), preferably several located in a distance of around 50 cm one from another, passing above the bottom of the biofilter, tangent to its horizontal section and having from down, along its entire length, holes (4), located in such a way, that the whole segment of the pipe located inside the biofilter is divided into five equal segments, the length of each being equal to 20% of the length of the pipe, and in the consecutive segments holes are made in the following order: on certain segment, starting from waste dump gas inlet, there is one row of holes, on the second segment there are two rows, on the third segment there are three rows, on the forth - four rows and on the fifth segment there five rows having a section area so selected, that the total area of the section of all holes (4) is equal to the internal area of the pipe (2a) and above the pipe (2a), in the biofilter (3) there is a grate (5) made of net with mesh diameter of 0,5 cm and on the grate (5) there is a layer (6) 5-15 cm thick, preferably 10 cm thick, of keramsite having a granularity of 0,6 to 1,2 cm, and above the keramsite layer (6) there is a 50-80 cm, preferably 70 cm high layer of organic material, preferably a layer of compost, in which layer methane oxidising bacteria can develop.

2. Device according to claim 1 **chrarcterised in that** the waste dump gas present in the biofilter in the space beneath the grate (5) attains constant overpressure, the same as that in the degassing well (1), which ensures its uniform inflow to the entire surface of the layers (6 and 7) in the biofilter (3)
